# EUROPEAN PATENT APPLICATION

(11) **EP 4 506 003 A1**
(43) Date of publication of application: **12.02.2025**
(21) Application number: 24192565.0
(22) Date of filing: 02.08.2024
(51) Int. Cl.: A61K 31/4709, A61P 35/02

(54) **HALOGEN DERIVATIVES OF (4´,5´- METHYLENEDIOXYPHENYL)-6,7-DIMETHOXY-1,2,3,4-TETRAHYDROISOQUINOLINE FOR USE IN THE TREATMENT OF LEUKEMIA**

(30) Priority: 07.08.2023 UZ 2300438
(71) Applicant: S.Yu Yunusov Institute of Chemistry of Plant Substances of the Academy of Sciences of the Republic of Uzbekistan, 100170 Tashkent (UZ); Republican Specialized Scientific Pratical Medical Center of Hematology of the Ministry of Public Health of the Republic of Uzbekistan, 100115 Tashkent (UZ)
(72) Inventor: Azimova, Shaxnoz Sadikovna, 100000 Tashkent (UZ); Terenteva, Yekaterina Olegovna, 100204 Tashkent (UZ); Xamidova, Umida Baxriddin Qizi, 100206 Tashkent (UZ); Umarova, Mukaddas Rustamovna, 100164 Tashkent (UZ); Tosheva, Nigora Arziyevna, 100204 Tashkent (UZ); Boboev, Kodirzhon Tukhtabaevich, 100115 Tashkent (UZ); Alimov, Timur Raufovich, 100123 Tashkent (UZ); Mustafina, Liya Kamilevna, 100115 Tashkent (UZ); Jo raqulov, Sherzod Niyatqobulovich, 100102 Tashkent (UZ); Saidov, Abdusalom Shomurodovich, 141627 Urgutskiy District, Ma . Sariktepa (UZ); Vinogradova, Valentina Ivanovna, 100200 Tashkent (UZ); Yusupova, Elvira Gaynatovna, 100124 Tashkent (UZ)
(74) Representative: Wirnsberger & Lerchbaum Patentanwälte OG

(57) **Abstract**

The use of compounds with the following structural formula is proposed: 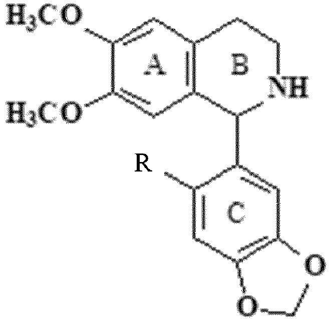 wherein R represents Br (compound F1) or Cl (compound F2),
as agents that have cytotoxic activity against CCRF-CEM cells (T-lymphoblastic leukemia) and exhibit an antitumor effect against various types of leukemia - T-acute lymphoblastic leukemia (T-ALL), B-acute lymphoblastic leukemia (B-ALL), acute lymphoblastic leukemia (ALL), acute myeloblastic leukemia (AML), acute myelomonoblastic leukemia (AMML and acute monocytic leukemia (AML).

## Description

The invention relates to the fields of chemistry, molecular biology, pharmaceuticals and medicine, exactly to substances exhibiting antitumor effects.

Over the past decades, the arsenal of antitumor drugs has expanded significantly, including isoquinoline alkaloids and their semisynthetic derivatives. Thus, the authors of patent RU 2413515 characterized isoquinolines with a phthalizine framework, which exhibit a cytotoxic effect on breast cancer cells [1], patent RU 2530775 describes 5-(2,6-difluorophenyl)-3-{[1-(methylsulfonyl)piperidine-4-yl]amino}-1H-pyrazolo[4,3-c]isoquinoline-7-carboxamide, effective against the cervix [2]. Isoquinolines have been characterized as demonstrating a high cytotoxic effect on tumor cells of the larynx, breast, and cervix [3,4].

Macrolide isoquinoline alkaloids cyclepeltin, zephyranthine and methyltelobin-N have pronounced cytostatic activity and inhibit the growth of cervical carcinoma cells in low concentrations [5]. Isoquinoline 5-(2,6-difluorophenyl)-3-{[1-(methylsulfonyl) piperidin-4-yl] amino}-1H-pyrazolo[4,3-c]isoquinoline -7-carboxamide also showed a pronounced antitumor effect on this type of cancer cells and the CE50 dose was 259 nM [6]. Various derivatives have also proven effective against breast cancer [7]. Thus, the authors present a wide range of isoquinolines and dihydroisoquinolines that exhibit cytotoxic effects [8]. 3N-(4-methylbenzoyl)- and 3N-(4-methoxybenzoyl)- derivatives of 3-benzoylamino-6,7-dimethoxy-1-methylisoquinoline are known in the literature, which exhibit high cytotoxic activity against CaOV ovarian carcinoma cells [9], and the tetrahydroisoquinoline alkaloid renieramycin M, isolated from the blue sponge *Xestospongia sp*, is active against lung cancer H460. The alkaloid shows high cytotoxic and antimetastatic activity with IC50=40 µM [10]. Tetrahydroisoquinolines are also active against glioblastoma, the most aggressive form of brain tumor, and carcinomas. Studies of the structure-function relationship have shown that the substance EDL-360 (12) is a powerful antiglioma agent against the LN18 cell line (IC50: 5.42 ± 0.06 µM), in which the functional group is the carbon linker between the biphenyl and the tetrahydroisoquinoline ring [11]. Scientists have found that the presence of alkyl substituents 1-cyclobutyl-, 1-cyclohexyl-, 1-phenyl or 1-benzylate at the C-1 position of the isoquinoline molecule causes significant death of pheochromocytoma of the adrenal medulla PC12 by triggering apoptosis [12, 13]. It is interesting to note that in the literature has information about tetrahydroisoquinolines that overcome multidrug resistance of cancer cells [15, 16, 17].

The experimental antitumor drug Pixantrone (Cell Therapeutics, USA), marketed under the brand name Pixuvri, contains the isoquinoline derivative 6,9-bis(2-aminoethylamino) benzoisoquinoline-5,10-dione as an active base [18].

The closest analogue to the claimed invention is 1-tridecyl-6,7-dimethoxy-1,2,3,4-tetrahydroisoquinoline, which has selective cytotoxicity against breast tumor lines HBL-100, T-lymphoblastic leukemia CCRF-CEM, skin melanoma LML, laryngeal adenocarcinoma Hep-2 and cervical carcinoma HeLa [14].

The objective of the invention is to expand the arsenal of agents that have cytotoxic activity against CCRF-CEM cells (T-lymphoblastic leukemia) and exhibit an antitumor effect against various types of leukemia - T-acute lymphoblastic leukemia (T-ALL), B-acute lymphoblastic leukemia (B -ALL), acute lymphoblastic leukemia (ALL), acute myeloblastic leukemia (AML), acute myelomonoblastic leukemia (AMML) and acute monocytic leukemia (AML).

The technical effect is that the proposed compounds have higher cytotoxicity towards cancer cells and lower toxicity (toxicity class 3) to the body, compared to known drugs used to treat leukemia.

The problem is solved by implementation of the compounds with the following structural formula: 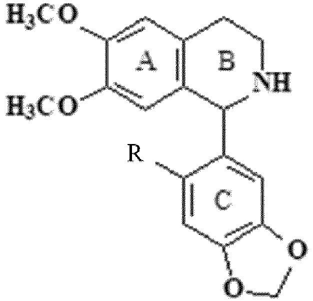 wherein R represents Br or Cl, as a drug that has cytotoxic activity against CCRF-CEM cells (T-lymphoblastic leukemia) and exhibits an antitumor effect against various types of leukemia - T-acute lymphoblastic leukemia (T-ALL), B-acute lymphoblastic leukemia (B-ALL), acute lymphoblastic leukemia (ALL), acute myeloblastic leukemia (AML), acute myelomonoblastic leukemia (AMML) and acute monocytic leukemia (AML).

In this case, the compound of the above structural formula is (1-(2'-bromo-4',5'-methylenedioxyphenyl)-6,7-dimethoxy-1,2,3,4-tetrahydroisoquinoline) (compound F1) and (1-(2'-chloro-4',5'-methylenedioxyphenyl)-6,7-dimethoxy-1,2,3,4-tetrahydroisoquinoline) (compound F2).

The term "antitumor effect" refers to a biological effect that can be manifested in various ways, including, but not limited to, for example, reduction in tumor volume, reduction in the number of tumor cells, reduction in the number of metastases, increase in life expectancy, decrease in tumor cell proliferation, decrease in survival tumor cells or improvement of various physiological symptoms associated with a malignant condition.

Compound F1 is known for use as an agent with cytotoxic activity against HeLa cervical cancer cells [19].

However, the cytotoxic activity of compounds F1 and F2 against CCRF-CEM cells is unknown in the state of the technique.

It is significant that compound (1-(2'-bromo-4',5'-methylenedioxyphenyl)-6,7-dimethoxy-1,2,3,4-tetrahydroisoquinoline) (compound F1) and compound (1-(2 '-chloro-4',5'-methylenedioxyphenyl)-6,7-dimethoxy-1,2,3,4-tetrahydroisoquinoline) (compound F2) have cytotoxic activity against CCRF-CEM cells, and also have a pronounced effect on myelocyte germ of hematopoiesis, as evidenced by its high efficiency in acute myeloid leukemia, acute monomyeloid leukemia and acute lymphocytic leukemia, while the LD₅₀ is 240.0±13.8 mg/kg (toxicity class 3), which is 57 times more than that of cytozar (cytarabine) and 22 times more than doxorubicin, that is, this compound is much less toxic than those used in the treatment of leukemia. Therefore, can be successfully used for the treatment of oncohematological diseases.

We offered compounds F1 and F2, in contrast to currently used anticancer drugs, which have a selective effect on cancer and healthy cells.

The above compounds F1 and F2 were synthesized according to the method described in documents [18,19], their cytotoxic effect was studied both on cancer and normal cell lines, and on blood samples from patients with leukemia: T- and B- acute lymphocytic leukemia, acute lymphocytic leukemia, acute myeloid leukemia, acute monocytic leukemia and acute myelomonoblastic leukemia.

The essence of the invention is illustrated by the following examples.

### Example 1. Synthesis of 1-(2-Bromo-4',5'-methylenedioxyphenyl)-6,7-dimethoxy-1,2,3,4-tetrahydroisoquinoline (F1)

A mixture of 3,4-dimethoxyphenylethylamine (1) (4.39 g, 24.25 mmol) and 2-bromo-4,5-methylenedioxybenzaldehyde (5.56 g, 24.27 mmol) (4) in 20 ml of trifluoroacetic acid was refluxed for 4 hours. The progress of the reaction was monitored by TLC. Next, the reaction mixture was cooled and alkalinized with ammonia to pH 9-10, then the amine was exhaustively extracted with chloroform. After distilling off the chloroform, the crude product was dissolved in acetone while heating and acidified with concentrated HCI to pH 5-6. The resulting hydrochloride precipitate was filtered and washed 3 times with acetone. We obtained (F1), with a yield of 8.35 g (94%), m.p. hydrochloride 201-203 °C (from CH3-CO-CH3), Rf 0.52 (chloroform-methanol system, 6:1) [20].

Mass spectrum (70eV) (m/z, Iots, %): 393 / 391 (M+, 100, Br-79/81), 379/377 (24, Br-79/81), 363 / 361 (14 /13, Br-79/81) 193 (22), 192 (65);
IR spectrum (KBr, vmax, cm⁻¹): 3424, 2967, 2840, 2562, 1674, 1516, 1479, 1445, 1393, 1374, 1306, 1285, 1215, 1129, 1034, 998, 927, 861, 84 1, 801, 762, 719; ¹H NMR (400 MHz, CDCl3, δ, ppm, J/Hz): 2.79 (1H, dt, J=5.9, 15.9, Na-4), 2.86 (1H, dt, J=5.9, 15.9, H_{b} -4), 3.02 (2H, ddd, J= 5.1, 6.6, 11.8, Na-3), 3.11 (1H, ddd, J= 5.1, 6.6, 11.8, H_{b}-3), 3.71 (3H, s, 7- OCH₃), 3.88 (3H, c, 6-OCH₃), 5.44 (1H, s, H-1), 5.93 (2H, dd, J= 1.3, 7.0, 4'-OCH₂O-5'), 6.27 (1H, s, H-8), 6.45 (1H, s, H-3'), 6.63 (1H, s, H-5), 7.04 (1H, s, H-6').

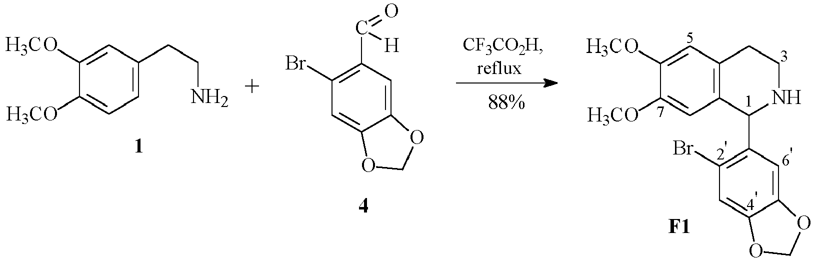

### Example 2. Synthesis of 1-(4',5'-methylenedioxy-2'-bromophenyl)-6,7-dimethoxy-1,2,3,4-tetrahydroisoquinoline (F1).

A solution of 4.20 g of 3,4-dimethoxyphenylethylamine (1) (23.20 mmol) and 4.31 g (23.23 mmol) of 2-chloro-4,5-methylenedioxybenzaldehyde (2) in 30 ml of benzene was boiled with azeotropic distillation of water using a Dean-Stark nozzle. The completion of the reaction was monitored by TLC. Benzene was distilled off, and the resulting imine was cyclized without purification by heating in a water bath in 15 ml of trifluoroacetic acid. The reaction mixture was cooled and basified with ammonia to pH 9-10, then the amine was exhaustively extracted with chloroform. After distilling off the chloroform, the crude product was dissolved in acetone and acidified with concentrated HCI to pH 5-6. The precipitated hydrochloride was separated. Yield 7.09 g (88%), mp. 107-108 °C (from CH₃-CO-CH₃), Rf 0.73 (chloroform-methanol system, 6:1) [20].

### Example 3. Determination of the cytotoxicity of compounds F1 and F2 on cell lines in vitro.

The cytotoxic properties of the compounds were determined *in vitro* using the MTT method [21] in 96-well plates (Costar, USA). Cell lines CCRF-CEM (CCL-119) (T-lymphoblastic leukemia) were cultured in RPMI-1640 and DMEM/12F medium (Himedia, India) containing 10% fetal bovine serum, 2 mM glutamine (Himedia, India) and 1 % antibiotic-antimycotic (Himedia, India) for 24 hours. Rat liver hepatocytes were obtained according to the method [22], human blood mononuclear cells - according to [23]. Fibroblast cells were obtained according to the method of [24] with some modifications. Cell cultures were subcultured into 96-well plates according to the method of [21] by adding 100 µl of nutrient medium. The CCRF-CEM suspension culture was poured into a tube, centrifuged at 800 rpm for 10 minutes, and the supernatant was discarded. Next, F1 and F2 were added to each well at a concentration of 1, 10, 100 µM, pre-dissolved in DMSO (no more than 0.8% by volume of the medium), and left in a CO₂ incubator (SHELLAB, USA) for 24 hours. During the incubation period, 20 µl of MTT reagent (5 mg/ml) was added to the samples and the optical density at 620 nm minus the measured background absorbance was determined using a 2300 EnSpire^{®} Multimode Plate Reader analyzer (PerkinElmer, USA). Data obtained in 3 independent experiments were expressed as the mean value for each concentration ± standard error of the mean, relative to the control values (cells without test compounds). The cytotoxic effect of each of the studied compounds was compared with the effect of the cytostatic Cisplatin-Naprod (India), containing cisplatin as an active component. The data is presented in Table 1.

**Table 1: IC₅₀ µM value for test compounds**

| 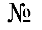 | Name of substances | Chemical formula | IC₅₀ | Cell sensitivity |
|---|---|---|---|---|
| 1 | 1-(2'-bromo-4',5'-methylenedioxyphen yl)-6,7-dimethoxy-1,2,3,4-tetrahydro isoquinoline (F1) | 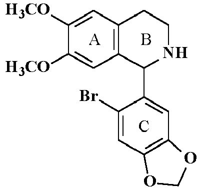 | 36.6 µM | CCRF-CEM |
| | | | >100 µM | fibroblasts |
| | | | >100 µM | hepatocytes |
| 2 | 1-(2'-chloro-4',5'-methylenedioxyphen yl)-6,7-dimethoxy-1,2,3,4-tetrahydro isoquinoline (F2) | 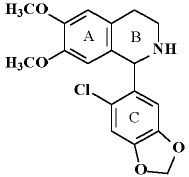 | 21.0 µM | CCRF-CEM |
| | | | >100 µM | fibroblasts |
| | | | >100 µM | hepatocytes |
| | Cisplatin | | 12,4µM | CCRF-CEM |
| | | | 29.4 µM | fibroblasts |
| | | | 28.7 µM | hepatocytes |

### Example 4 Acute Toxicity Study (LD₅₀) of Compound F1

We studied the acute toxicity of compound F1 *in vivo.* The experiments were carried out on outbred white mice of both sexes weighing 18-20 g (24 pcs.). The animals were divided into groups of 12 mice each. The compound was dissolved in DMSO and then adjusted to the desired concentration with 0.9% NaCl solution and administered intraperitoneally and subcutaneously in a dose range from 5.0 to 200.0 mg/kg. The animals were observed for a week. The maximum relative error of the applied dose was no more than 5%.

Table 2 shows the "dose-toxicity" values for compound F1

**Table 2: Determination of LD₅₀ dose for F1**

| | LD₅₀ dose, mg/kg (intraperitoneal) |
|---|---|
| F1 | 240,0±13,8 |
| cytarabine* | 4.2±0,5 |
| doxorubicin** | 10,8±1,0 |

The death of animals at doses of 240.0±13.8 occurred on days 3-5 of the experiment. With an increase in F1 concentration, death occurred within 2 days. Before death, the animals exhibited weak motor activity and lack of appetite. The LD₅₀ doses we established for cytarabine and doxorubicin correspond to literature data [25]*, [26]**.

The LD₅₀ value of compound F1 was found to be 240.0±13.8 mg/kg. According to these data, compound F1 can be classified as toxicity class 3 (moderately toxic) [27].

### Example 5. Study of acute toxicity (LD₅₀) of compound F2

We studied the acute toxicity of compound F2 *in vivo.* The experiments were carried out on outbred white mice of both sexes weighing 18-20 g (24 pcs.). The animals were divided into groups of 12 mice each. The compound was dissolved in DMSO and then brought to the desired concentration with 0.9% NaCl solution and administered intraperitoneally and subcutaneously to animals in a dose range from 5.0 to 200.0 mg/kg. The animals were observed for a week. The maximum relative error of the administered dose was no more than 5%.

The first 24 hours were critical for the toxic effect to occur. Table 3 shows the dose-toxicity values for compound F2

**Table 3: Determination of LD₅₀ dose for F2**

| | LD₅₀ dose, mg/kg (intraperitoneal) |
|---|---|
| F1 | 220,0±15,6 |
| cytarabine* | 4.2±0,5 |
| doxorubicin** | 10,8±1,0 |

The death of animals at doses of 220.0±15.6 occurred on days 3-5 of the experiment. With an increase in F2 concentration, the death of animals occurred within 2 days. Before death, the animals exhibited weak motor activity and lack of appetite. The LD50 doses we established for cytarabine and doxorubicin correspond to literature data [25]*, [26]**.

It was found that the LD₅₀ value of compound F2 was 220.0±15.6 mg/kg. According to these data, compound F2 can be classified as toxicity class 3 (moderately toxic) [27].

### Example 6. Study of the effect of compounds F1, F2 on hemograms of blood samples from patients with leukemia

The effect of compounds F1 and F2 was studied on biological material (blood) of patients with hematological malignancies (leukemia), as well as for individual nosologies, in particular, patients with leukemia (combined group) (n=224), T-ALL (n=84), B-ALL (n=100), ALL (n=100), AML (n= 98), AML (n=94) and AML (n= 98) were studied. A total of 798 blood samples from patients with leukemia were examined.

The drugs Cytosar (Alexan) (Ebewe Pharma, Austria) and doxorubicin (Naprod, Mumbai, India) recommended by WHO for the treatment of the above oncohematological diseases were used as comparison drugs.

5 ml of venous blood from sick and conditionally healthy individuals were collected into Vacutainer tubes with the addition of EDTA (Himedia, India).

The resulting blood was divided into aliquots of 1 ml and 10 µl (10 µg/ml) of the studied drugs (cytosar, doxorubicin, F1 and F2) were added to each tube.

Then, after 3 hours of incubation at a temperature of 37.0°C, smears were prepared according to the generally accepted method [28], stained and blood morphology was examined according to the standard method [29].

### Example 7. Study of the effect of compounds F1 and F2, cytosar, doxorubicin on the hemogram of conditionally healthy individuals.

The study was carried out as described in example 6.

**Table 4: Hemogram of conditionally healthy individuals after exposure to compounds F1 and F2**

| Parameters, % | Control, n=32 | Comparator preparations | | Main groups | |
|---|---|---|---|---|---|
| | | Cytosar, n=49 | Doxorubicin, n=41 | Compound F1, n=54 | Compound F2, n=55 |
| Blasts | 0,0±0,0 | 0,0±0,0 | 0,0±0,0 | 0,0±0,0 | 0,0±0,0 |
| Destroyed cells | 0,0±0,0 | 0,0±0,0 | 0,0±0,0 | 0,0±0,0 | 0,0±0,0 |
| Myelocytes | 0,0±0,0 | 0,0±0,0 | 0,0±0,0 | 0,0±0,0 | 0,0±0,0 |
| Promyelocytes | 0,0±0,0 | 0,0±0,0 | 0,0±0,0 | 0,0±0,0 | 0,0±0,0 |
| Band (s/n) neutrophils | 1,8±0,2 | 1,8±0,2* | 1,5±0,2 | 1,5±0,2* | 1,6±0,2 |
| Segmented (s/i) neutrophils | 64,1±1,2 | 61,5±1,2 | 62,6±1,0 | 62,6±1,0 | 59,4±2,8 |
| Eosinophils | 1,6±0,2 | 2,1±0,4 | 1,7±0,2 | 1,7±0,2 | 1,7±0,3 |
| Lymphocytes | 26,3±1,2 | 28,0±1,2 | 28,1±0,9 | 28,1±0,9 | 26,9±1,6 |
| Monocytes | 6,2±0,4 | 6,6±0,4* | 6,1±0,4 | 6,1±0,4* | 5,1±0,4 |

| | | | | | |
|---|---|---|---|---|---|
| Note:* - statistically significant differences (p<0.05) compared to the control group; ^ - - statistically significant differences (p<0.05) compared with the cytosar group; # - statistically significant differences (p<0.05) compared to doxorubicin. | | | | | |

A comparison in a group of relatively healthy individuals showed that the effect of compounds F1 and F2 on the blood does not lead to significant differences in hemogram parameters, which indicates their safety.

### Example 8. Effect of compounds F1 and F2 on the hemogram of patients with an established diagnosis of leukemia.

The study was carried out as in example 6.

**Table 5: Hemogram of patients with leukemia after exposure to compounds F1 and F2**

| Parameters, % | Control, n=25 | Comparator preparations | | Main groups | |
|---|---|---|---|---|---|
| | | Cytosar, n=49 | Doxorubici n, =41 | Compound F1, n=54 | Compoun d F2, n=55 |
| Blasts | 77,1±2,9 | 65,9±2,8* | 65,6±2,6* | 65,7±2,3* | 65,2±2,8* |
| Destroyed cells | 2,9±0,7 | 9,0±1,6* | 11,4±2,2* | 14,8±1,8*^ # | 12,9±1,8* |
| Myelocytes | 0,3±0,1 | 1,1±0,3* | 3,3±1,2 | 4,0±0,5^# | 2,5±0,6* |
| Promyelocytes | 0,0±0,0 | 0,2±0,1 | 0,0±0,000 | 5,0±0,6^ | 3,8±2,1 |
| Band (s/n) neutrophils | 0,6±0,2 | 1,1±0,4* | 1,8±0,5 | 2,5±0,3*^# | 1,6±0,2* |
| Segmented (s/i) neutrophils | 4,7±0,8 | 6,7±1,1 | 5,8±0,7 | 7,0±0,8 | 5,5±0,9 |
| Eosinophils | 0,1±0,1 | 0,4±0,3 | 3,8±2,4 | 4,0±0,6^# | 1,6±0,3* |
| Lymphocytes | 12,7±2,0 | 13,7±1,6 | 15,0±1,8 | 12,0±0,9 | 12,3±1,2 |
| Monocytes | 1,7±0,5 | 3,5±0,6* | 5,3±1,2 | 4,9±0,6* | 4,7±0,7* |

| | | | | | |
|---|---|---|---|---|---|
| Note: the same as Table 4. | | | | | |

Studies have shown that doxorubicin and compound F2 reduce the number of blasts.

As can be seen from Table 5, compound F1 statistically significantly reduces the content of blasts by 10.7% (p<0.05), which inversely correlates with an increase in the proportion of destroyed cells by 9.3% (p<0.05) (Table 2). Another positive aspect was the increase in the proportion of more mature cellular elements: an increase in the percentage of neutrophils by 1.8 times (p<0.05). There was also a pronounced tendency to increase the proportion of neutrophils. The proportion of eosinophils, which are mature, more differentiated elements of the leukocyte formula, increased statistically significantly by 2.7 times (p<0.05). All the described changes indicate a positive effect of compound F1 on the hemogram and indicate its potential effectiveness in leukemia.

The study showed that compound F2 led to a decrease in the level of blast cells by 14.6% lower than in the "Control" group. There was also a 2.3-fold increase in the level of destroyed cells after exposure to compound F2 compared to the control group. These results indicate the effectiveness of this compound when affecting the peripheral blood of patients with leukemia.

The presented results show that after the use of compound F2, most hematological parameters do not have statistically significant differences from the groups where PC (peripheral blood) was exposed to cytosar and doxorubicin, which indicates that the test compound is not inferior in effectiveness to drugs traditionally used in clinical practice.

The use of compound F 1, according to the hemogram, showed an increase in the % of destroyed cells, and also led to an increase in the proportion of such mature differentiated cells of the leukocyte series as sub-cells and sub-neutrophils.

Summarizing the above-described data, we can conclude that in leukemia, in general, the most effective effect was shown by compound F1, which was expressed in the greatest decrease in the percentage of blasts, an increase in the proportion of destroyed cells, a high content of relatively more mature cells such as myelocytes, promyelocytes and neutrophils, eosinophils and monocytes (Table 5).

It was found that compound F1 at the same concentrations is more effective in its action than cytosar and doxorubicin, with the LD50 value being 57 times and 22 times higher than for the reference drugs, respectively. Compound F2 is comparable in its effect to cytosar and doxorubicin, and the LD₅₀ value is 52 and 20 times higher than for the reference drugs, respectively.

### Example 9. Effect of compounds F1 and F2 on the hemogram of patients with T-ALL

The study was carried out in accordance with the description given in example 5.

**Table 6: Hemogram of patients with T-ALL after exposure to compounds F 1 and F2**

| Parameters, % | Control, n=16 | Comparator preparations | | Main groups | |
|---|---|---|---|---|---|
| | | Cytosar, n=16 | Doxorubici n, =18 | Compound F1, n=16 | Compoun d F2, n=18 |
| Blasts | 80,3±1,3 | 73,5±2,0* | 67,2±0,9* | 68,9±1,7* | 68,2±2,2* |
| Destroyed cells | 4,9±0,6 | 8,8±1,0* | 13,4±0,5* | 16,9±1,1*^ # | 16,1±1,3* A |
| Myelocytes | 0,0±0,0 | 0,0±0,0 | 0,0±0,0 | 0,0±0,0 | 0,0±0,0 |
| Promyelocytes | 0,0±0,0 | 0,0±0,000 | 0,0±0,0 | 0,0±0,0 | 0,0±0,000 |
| Band (s/n) neutrophils | 1,1±0,2 | 1,0±0,1 | 1,2±0,1* | 0,0±0,0 | 0,8±0,2 |
| Segmented (s/i) neutrophils | 4,1±0,5 | 3,0±0,4* | 6,7±0,4 | 3.9±0,4# | 7,0±1,0*^ |
| Eosinophils | 0,0±0,0 | 0,0±0,0 | 0,0±0,0 | 0,0±0,6 | 0,0±0,0 |
| Lymphocytes | 8,6±0,8 | 11,5±1,0* | 9,8±0,1* | 9,1±1,0 | 6,7±0,6*^ # |
| Monocytes | 1,0±0,2 | 2,3±0,4 | 1.7±0,2 | 1,3±0,3* | 1,2±0,2*^ |

| | | | | | |
|---|---|---|---|---|---|
| Note: the same as Table 4. | | | | | |

A comparative study showed the presence of statistically significant differences after the use of F1, the main of which was a statistically significant increase in the proportion of destroyed cells by 1.9 times (p<0.001) and 1.3 times (p<0.01), compared with cytosar and doxorubicin respectively. After using F1, the percentage of p/i neutrophils decreased to 0.0±0.0, and p/i neutrophils - by 1.7 times (p<0.001).

After application of F2, a pronounced tendency towards a decrease in blasts was observed, correlating with a statistically significant increase in the percentage of destroyed cells by 1.8 times (p<0.001). Comparison with the cytosar group showed that the proportion of mature and differentiated neutrophils after F2 increased 2.3 times (p≤0.05). Lymphocytes decreased statistically significantly by 1.7 times (p<0.001), compared with doxorubicin, as did monocytes, which decreased by 1.9 times (p<0.02).

Comparison of the results of the effects of the studied compound F2 relative to doxorubicin showed a pronounced tendency towards an increase in the proportion of destroyed cells and a tendency towards a decrease in the proportion of immature granulocytes - neutrophils. The proportion of lymphocytes decreased statistically significantly compared to the group in which doxorubicin was used.

For T-ALL, the most effective effect was demonstrated by compound F2 compared to cytosar and doxorubicin, which was expressed in the greatest decrease in the percentage of blasts, an increase in the proportion of destroyed cells, and a high content of relatively more mature cells such as myelocytes, promyelocytes and subcutaneous neutrophils, eosinophils and monocytes (Table 6).

### Example 10. Effect of compounds F1 and F2 on the hemogram of patients with B-ALL

The study was carried out in accordance with the description given in example 5.

**Table 7: Hemograms of patients with B-ALL after exposure to F1 and F2**

| Parameters, % | Control, n=20 | Comparator preparations | | Main groups | |
|---|---|---|---|---|---|
| | | Cytosar, n=20 | Doxorubici n, =20 | Compound F1, n=20 | Compound F2, n=20 |
| Blasts | 68,4±3,2 | 63,5±3,4* | 64,3±3,7 | 59,0±2,9* | 53,9±2,6*^# |
| Destroyed cells | 3,1±0,8 | 8,9±2,2* | 13,0±2,8* | 12,6±2,0* | 15,2±1,8*^ |
| Myelocytes | 0,0±0,0 | 0,7±0,4 | 0,6±0,3 | 0,5±0,2 | 0,8±0,2 |
| Promyelocytes | 0,0±0,0 | 0,0±0,0 | 0,0±0,0 | 0,0±0,0 | 0,0±0,0 |
| Band (s/n) neutrophils | 1,1±0,3 | 0,6±0,3 | 0,7±0,2 | 1,4±0,4 | 1,7±0,4 |
| Segmented (s/i) neutrophils | 4,6±0,9 | 6,1±0,9 | 4,9±0,9 | 9,4±0,9* | 11,1±2,0* |
| Eosinophils | 0,4±0,2 | 0,1±0,1 | 0,1±0,1 | 0,2±0,1 | 0,0±0,0 |
| Lymphocytes | 19,9±2,6 | 17,2±2,0 | 15,0±2,9 | 13,5±1,7* | 14,6±2,0 |
| Monocytes | 3,1±0,7 | 4,6±1,2 | 1.6±0,5 | 3,5±0,8# | 2,8±0,9 |

| | | | | | |
|---|---|---|---|---|---|
| Note: the same as Table 4. | | | | | |

A study of the effectiveness of F1 showed no statistically significant differences in comparison with cytosar and doxorubicin, which indicates that the effectiveness of this compound in B-ALL is not inferior to well-known comparator drugs widely used in clinical practice.

A study of F2 compared with cytosar and doxorubicin showed statistically significant differences. Thus, after the use of F2, a more pronounced, statistically significant decrease in the content of blasts was observed by 1.27 times (p<0.05), while after the use of F1 - by 1.16 times. The proportion of destroyed cells after application of F2 increases - 4.9 times (p<0.05), and after F1 - 4.1 times (p<0.05).

In B-ALL, compound F2 showed the most effective effect compared to cytosar and doxorubicin. Compound F1 is comparable in level of impact to the comparator drugs cytosar and doxorubicin.

### Example 11. Effect of compounds F1 and F2 on the hemogram of patients with ALL

The study was carried out in accordance with the description given in example 5.

**Table 8: Hemograms of patients with ALL after exposure to F1 and F2**

| Parameters, % | Control, n=20 | Comparator preparations | | Main groups | |
|---|---|---|---|---|---|
| | | Cytosar, n=20 | Doxorubici n, =20 | Compound F1, n=20 | Compound F2, n=20 |
| Blasts | 88,6±0,8 | 78,7±1,4* | 76,4±1,7* | 68,4±2,7*^ | 67,9±1,6*^ |
| Destroyed cells | 0,8±0,2 | 4,8±1,4* | 9,0±1,1*^ | 12,9±1,7*^ | 13,2±1,4*^ # |
| Myelocytes | 0,0±0,0 | 0,2±0,1 | 0,2±0,1 | 0,0±0,0 | 0,0±0,0 |
| Promyelocytes | 0,0±0,0 | 0,0±0,0 | 0,0±0,0 | 0,0±0,0 | 0,0±0,0 |
| Band (s/n) neutrophils | 0,2±0,1 | 0,4±0,2 | 0,3±0,1 | 0,3±0,1 | 0,7±0,2 |
| Segmented (s/i) neutrophils | 1,2±0,1 | 2,4±0,6 | 2,2±0,3 | 4,2±1,0* | 3,7±0,7* |
| Eosinophils | 0,0±0,0 | 0,0±0,0 | 0,2±0,1 | 0,0±0,0 | 0,2±0,1 |
| Lymphocytes | 8,8±0,8 | 11,4±0,8 | 9,9±1,3 | 12,6±1,1* | 13,4±1,3 |
| Monocytes | 0,4±0,2 | 2,2±0,4 | 1.9±0,6 | 1,8±0,4 | 1,1±0,3 |

| | | | | | |
|---|---|---|---|---|---|
| Note: the same as Table 4. | | | | | |

Compared with cytosar, statistically significant differences were revealed in the level of blast cells both after the use of F1 and after F2 - 1.15 times (p<0.05) and 1.16 times (p<0.05), respectively. There were statistically significant differences compared to cytosar in the percentage of destroyed cells, which was higher after the introduction of F1 and F2 - 2.7 times (p<0.05) and 2.8 times (p<0.05), respectively.

For compound F2, statistically significant changes were also observed in comparison with the effect of doxorubicin. Thus, the percentage of blasts was statistically significantly lower by 1.12 times (by 8.5%), and the proportion of destroyed cells increased by 2.75 times

According to a study in ALL, compound F2 showed the greatest effectiveness compared to the control group.

Also, after using F2, statistically significant differences were revealed in the percentage of destroyed cells compared to cytosar and doxorubicin, which was not observed after using F1.

### Example 12. Effect of compounds F1 and F2 on the hemogram of patients with AML

The study was carried out in accordance with the description given in example 5.

The study showed a decrease in the percentage of blasts both after the use of F1 and after F2 - 1.2 times, comparable to the number of blasts under the influence of cytosar and doxorubicin.

The level of destroyed cells increased by 4.5 times (p<0.05) and 4.0 times (p<0.05) after application of F1 and F2, respectively. The proportion of mature differentiated neutrophils increased after the use of F1 and F2 by 2.6 and 2.0 times (p<0.05), respectively.

**Table 9: Hemograms of AML patients after exposure to F 1 and F2**

| Parameters, % | Control, n=17 | Comparator preparations | | Main groups | |
|---|---|---|---|---|---|
| | | Cytosar, n=20 | Doxorubici n, =17 | Compound F 1, n=22 | Compound F2, n=22 |
| Blasts | 76,3±2,6 | 65,4±3,9* | 68,4±3,6* | 63,6±2,3* | 65,6±2,4* |
| Destroyed cells | 2,6±0,9 | 6,3±1,7* | 5,6±1,4* | 12,7±1,2*^ # | 8,2±1,1 |
| Myelocytes | 0,4±0,2 | 1,4±0,6 | 0,9±0,8 | 1,2±0,6 | 1,3±0,7 |
| Promyelocytes | 0,0±0,0 | 0,9±0,6 | 0,0±0,0 | 0,3±0,2 | 0,4±0,2 |
| Band (s/n) neutrophils | 0,7±0,2 | 1,2±0,5 | 0,5±0,4 | 1,2±0,3 | 0,9±0,4 |
| Segmented (s/i) neutrophils | 3,2±0,7 | 5,6±1,2 | 5,5±1,2 | 7,0±0,7* | 7,0±1,1* |
| Eosinophils | 1,2±0,7 | 1,0±0,9 | 0,8±0,7 | 0,4±0,2 | 0,8±0,5 |
| Lymphocytes | 12,2±1,0 | 12,5±1,7 | 13,2±2,4 | 10,7±1,1 | 11,5±1,8 |
| Monocytes | 3,9±1,0 | 3,4±0,8 | 5.2±1,5 | 3,0±0,7 | 4,4±0,9 |

| | | | | | |
|---|---|---|---|---|---|
| Note: the same as Table 4. | | | | | |

In general, the most pronounced and statistically significant positive changes in the composition of the leukocyte formula were observed precisely with the use of F 1, compared with the result achieved after using F2.

In AML, compared with cytosar, after using F1, an increase in the percentage of destroyed cells was found to increase by 2.0 times (p<0.01). The use of F1 compared with doxorubicin showed a statistically significant decrease in the percentage of destroyed cells in hemograms by 2.3 times (p<0.01).

In AML, comparison of hemogram parameters with cytosar and doxorubicin after the use of F2 showed the absence of statistically significant differences. Almost all hemogram parameters were at a comparable level with the results after using comparison drugs.

Compared to cytosar and doxorubicin, the most pronounced and statistically significant changes were observed after the use of compound F1.

### Example 13. Effect of compounds F1 and F2 on the hemogram of patients with AML

The study was carried out in accordance with the description given in example 5.

The use of F1 led to a decrease in the proportion of blasts by 1.6% (p<0.05), an increase in the proportion of destroyed cells by 5.3 times (p<0.05).

It was found that the content of blasts in the blood of patients with AML decreased after the use of F2 in the main group by 11.7% (p<0.01). At the same time, the percentage of destroyed cells increased by 2.6 times.

**Table 10: Hemograms of patients with AML after exposure to F1 and F2**

| Parameters, % | Control, n=17 | Comparator preparations | | Main groups | |
|---|---|---|---|---|---|
| | | Cytosar, n=20 | Doxorubici n, =18 | Compound F1, n=20 | Compound F2, n=19 |
| Blasts | 56,8±4,3 | 50,2±2,8 | 58,4±1,9 | 51,1±2,2 | 51,9±2,2 |
| Destroyed cells | 3,5±0,6 | 7,8±0,6* | 15,1±1,1* | 18,7±1,3*^ # | 16,6±1,6*^ |
| Myelocytes | 6,6±1,0 | 16,0±1,4* | 4,0±0,4* | 2,6±0,5*^# | 3,2±0,5* |
| Promyelocytes | 0,0±0,0 | 0,0±0,0 | 0,0±0,0 | 0,0±0,0 | 0,0±0,0 |
| Band (s/n) neutrophils | 3,1±0,6 | 3,6±0,5 | 3,4±0,5 | 2,8±0,3 | 2,9±0,4 |
| Segmented (s/i) neutrophils | 8,1±1,1 | 14,1±1,2* | 10,8±1,0 | 16,6±1,3*# | 18,6±1,7*# |
| Eosinophils | 0,2±0,1 | 0,6±0,3 | 0,7±0,3 | 0,8±0,3 | 0,5±0,2 |
| Lymphocytes | 8,6±1,1 | 6,6±0,7 | 6,1±0,7 | 6,2±0,6 | 3,2±0,8* |
| Monocytes | 8,1±1,0 | 1,2±0,2* | 1.6±0,3* | 1,4±0,2* | 3,2±0,4* |

| | | | | | |
|---|---|---|---|---|---|
| Note: the same as Table 4. | | | | | |

Also, under the influence of the studied compounds, the ratio between immature cellular elements (blasts), intermediate forms (promyelocytes, myelocytes) and representatives of the "normal" leukoformula changed. There was a decrease in the percentage of myelocytes characteristic of AML by 2.5 times (p<0.05) and 2.1 times (p<0.05) after the use of F1 and F2, respectively. The proportion of neutrophils increased by 2.0 times (p<0.05) and 2.3 times (p<0.05) after exposure to F1 and F2, respectively.

Analysis of the blood hemogram of patients with AML showed an increase in the proportion of destroyed cells after the use of F1 by 2.4 times (p<0.01), compared with the results of using Cytosar. After F1, the percentage of myelocytes was 6.15 times lower (p<0.01) relative to cytosar.

Compared with the effect of doxorubicin, after F1 the proportion of destroyed cells was 1.2 times higher (p<0.05), myelocytes were 1.5 times lower (p<0.05), and neutrophils were 1 times higher .5 times (p<0.05).

The data obtained showed that in OMML after using F2, the percentage of destroyed cells was 2.1 times higher (p<0.01), and the percentage of myelocytes was 5.0 times lower (p<0.01), the percentage of lymphocytes was 2 times lower .1 times (p<0.01) than after using Cytosar.

Compared with the effect of using doxorubicin, after using F2, the percentage of neutrophils was 1.7 times lower (p<0.05), and the percentage of lymphocytes was 1.9 times lower (p<0.05).

Thus, in general, F1 had a more pronounced effect on the hemogram composition in AML. Compound F2 is comparable in level of impact to the comparator drugs cytosar and doxorubicin.

### Example 14. Effect of compounds F1 and F2 on the hemogram of patients with AML

The study was carried out in accordance with the description given in example 6

**Table 11: Hemograms of patients with AML after exposure to F1 and F2**

| Parameters, % | Control, n=18 | Comparator preparations | | Main groups | |
|---|---|---|---|---|---|
| | | Cytosar, n=20 | Doxorubici n, =20 | Compound F1, n=20 | Compound F2, n=20 |
| Blasts | 70,8±0,9 | 59,5±3,7* | 45,4±1,9* | 55,4±3,1* | 59,1±3,3* |
| Destroyed cells | 7,4±0,6 | 12,4±1,7* | 24,0±4,0* | 22,6±2,3*^ | 19,3±1,7* |
| Myelocytes | 1,5±0,2 | 1,8±0,4 | 0,0±0,0 | 0,9±0,3 | 0,4±0,1 |
| Promyelocytes | 0,0±0,0 | 0,0±0,0 | 0,0±0,0 | 0,0±0,0 | 0,0±0,0 |
| Band (s/n) neutrophils | 1,3±0,2 | 3,5±1,0* | 0,8±0,2 | 1,6±0,5 | 1,2±0,3 |
| Segmented (s/i) neutrophils | 8,0±0,6 | 9,2±2,3 | 9,1±1,5 | 9,6±1,7 | 9,6±2,5 |
| Eosinophils | 0,0±0,0 | 4,0±0,2 | 0,0±0,0 | 0,0±0,0 | 0,5±0,2 |
| Lymphocytes | 9,7±0,8 | 8,4 ±0,9 | 9,7±1,5 | 10,1±1,0 | 8,2±1,1 |
| Monocytes | 1,4±0,2 | 4,9±1,2* | 11.2±2,3* | 2.0±0,6^ | 1,8±0,3 |

| | | | | | |
|---|---|---|---|---|---|
| Note: the same as Table 4. | | | | | |

When studying the effect of compound F1 on the blood of patients with AML, in general, more pronounced positive changes in the composition of the leukocyte formula were observed: a more significant decrease in the proportion of blasts, an increase in the proportion of destroyed cells, a more significant increase in the percentage of s/c and s/c neutrophils, which are more mature, differentiated representatives of the leukocyte formula.

A study in AML showed that compared to Cytosar, F1 was more effective, as evidenced by the percentage of blasts, which was 4.1% lower after using this compound. In terms of certain indicators, in particular, in terms of cytotoxicity, it is even more effective - the percentage of destroyed cells was statistically significantly higher after the use of F1 than after the drug Cytosar - 1.8 times (p<0.05). The percentage of monocytes after F1 was 2.4 times lower (p<0.05) compared to the effect of cytosar.

After application of F1 and F2, relative to the doxorubicin group, no statistically significant differences were detected.

### Conclusion:

In case of AML, in comparison with cytosar, the most effective compound can be considered compound F1.

Compared to doxorubicin, compound F1 had no statistically significant differences, indicating a comparable level of effectiveness.

The effectiveness of F2 is comparable to both cytosar and doxorubicin.

At the same time, compound F1 has a more pronounced effect on the myelocytic lineage of hematopoiesis, as evidenced by its high effectiveness in AML, AML and AML.

Compound F2 has a pronounced effect on the lymphoid lineage of hematopoiesis, as evidenced by its high effectiveness in T-ALL, B-ALL and ALL.

Thus, the data presented show that compounds F1 and F2 have cytotoxic activity against CCRF-CEM cells, and also have a pronounced effect on the myelocytic lineage of hematopoiesis, as evidenced by their high efficiency in AML, OMML and AML, with an LD₅₀ of 240, 0±13.8 mg/kg (toxicity class 3), which is 57 times more than cytazar and 22 times more than doxorubicin, that is, this compound is much less toxic than those used in the treatment of leukemia. Therefore, it can be successfully used for the treatment of oncohematological diseases.

The present invention expands the arsenal of agents that have cytotoxic activity against CCRF-CEM cells and exhibit an antitumor effect against various types of leukemia - T-ALL, B-ALL, ALL, AML, OMMoL and OMoL, can be successfully used for the treatment of oncohematological diseases.

### References

1. RU 2413515
2. RU 2530775
3. UZ IAP 05683
4. UZ IAP 05664
5. Kartsev V.G., Tolstikova G.A., Nitrogen heterocycles and alkaloids, book, Moscow, 2001, p.97-98
6. RU 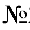 2530775
7. Lin Y., Shi R., Wang X., Shen H.M. Curr Cancer Drug Targets. Luteolin, a flavonoid with potential for cancer prevention and therapy. 2008; 8(7), 634-646. doi:10.2174/156800908786241050
8. RU 2490260
9. Lapa G.B. Study of the synthesis pathways of isoquinoline alkaloids and bisindole bases of Catharanthus roseus possessing cytostatic activity. Abstract of dissertation,1995
10. Halim H., Chunhacha P., Suwanborirux K., Chanvorachote P. Anticancer and antimetastatic activities of Renieramycin M, a marine tetrahydroisoquinoline alkaloid, in human non-small cell lung cancer cells. Anticancer Res, 2011, 31(1), 193-201.
11. Patil R., Hosni-Ahmed A., Jones T.S., Patil S.A., Asres L.B., Wang X., Yates R.C., Geisert E.E., Miller D.D. Synthesis and in vitro evaluation of novel. 1,2,3,4-tetrahydroisoquinoline derivatives as potent antiglioma agents. Anticancer Agents Med Chem, 2014, 14(3), 473-482. doi: 10.2174/1871520621666210112122913
12.Saitoh T., Abe K., Ishikawa M., Nakatani M., Shimazu S., Satoh N., Yoneda F.,Taguchi K., Hor iguchi Y. Synthesis and in vitro cytotoxicity of 1,2,3,4-tetrahydroisoquinoline derivatives. Eur J Med Chem, 2006, 41, 241-253. doi: 10.1016/j.ejmech.2005.11.003
13. Hatano H., Takekawa F., Hashimoto K., Ishihara M., Kawase M., Qing C., Qin-Tao W., Sakagami H. Tumor-Specific Cytotoxic Activity Of 1,2,3,4-Tetrahydroisoquinoline Derivatives Against Human Oral Squamous Cell Carcinoma Cell Lines// Anticancer Research, 2009, 29, p.3079-3086.
14. UZ 06621 IAP
15. Fang W., Li Y., Cai Y., Kang K., Yan F., Liu G., Huang W. Substituted tetrahydroisoquinoline compounds B3 inhibited P glycoprotein-mediated multidrug resistance in vitro and in vivo. J Pharm Pharmacol, 2007, 59, 1649-1655. doi: 10.1211/jpp.59.12.0006
16. Li Y., Zhang H.B., Huang W.L., Li Y.M. Design and synthesis of tetrahydroisoquinoline derivatives as potential multidrug resistance reversal agents in cancer. Bioorg Med Chem Lett, 2008, 18, 3652-3655. doi: 10.1016/j.bmcl.2008.04.082
17. Yan F., Jiang Y., Li Y-M., Zhen X., Cen J., Fang W-R. Reversal of P-glycoprotein and multidrug resistance-associated protein 1-mediated multidrug resistance in cancer cells by HZ08 isomers, tetraisohydroquinoline derivatives. Biol Pharm Bull, 2008, 31, 1258-1264. doi:10.1248/bpb.31.1258
18. www.drugbank.ca
19. UZ 05766 IAP
20. Zhurakulov Sh. N., Vinogradova V. I., LevkovichM. G. Synthesis of 1-aryltetrahydroisoquinoline alkaloids and their analogs. Chemistry of natural compounds. New York 2013. 49 (1), 70-74
21. Niks M., Otto M. Towards an optimized MTT assay. Journal of Immunological Methods, 1990, 130, 149-151
22. Cabral, F., Miller, C.M., Kudrna, K.M., Hass, B.E., Daubendiek, J.G., Kellar, B.M., Harris, E.N. Purification of Hepatocytes and Sinusoidal Endothelial Cells from Mouse Liver Perfusion. J. Vis. Exp. 2018. (132), e56993, doi:10.3791/56993
23. Kleiveland, C. R. (2015). Peripheral Blood Mononuclear Cells. The Impact of Food Bioactives on Health, 161-167. doi:10.1007/978-3-319-16104-4_15
24. Khamidova U.B., Terenteva E.O., Azimova Sh.S. Isolation of skin fibroblasts by new modified method. Uzbek biological journal. Tashkent. 2021, (5), 7-10
25. https://cdn.caymanchem.com/cdn/msds/16069m.pdf
26. B. Arechabala. J. Appl. Toxicol. Comparison of Cytotoxicity of Various Surfactants Testedon Normal Human Fibroblast Cultures using the Neutral Red Test, MTT Assay and LDH Release. France. 1999,19, 163-165
27. Wilson, Anne P. Chapter 7: Cytotoxicity and viability. Animal Cell Culture: A Practical Approach / Masters, John R.W. - 3rd. - Oxford: Oxford University Press, 2000. (1) - ISBN 978-0-19-963796-6.
28. Chernenok V. V. et al. Clinical laboratory blood tests. Indicators in norm and pathology. 2011
29. Trukhachev V. I. et al. Method of staining blood smears. 2007

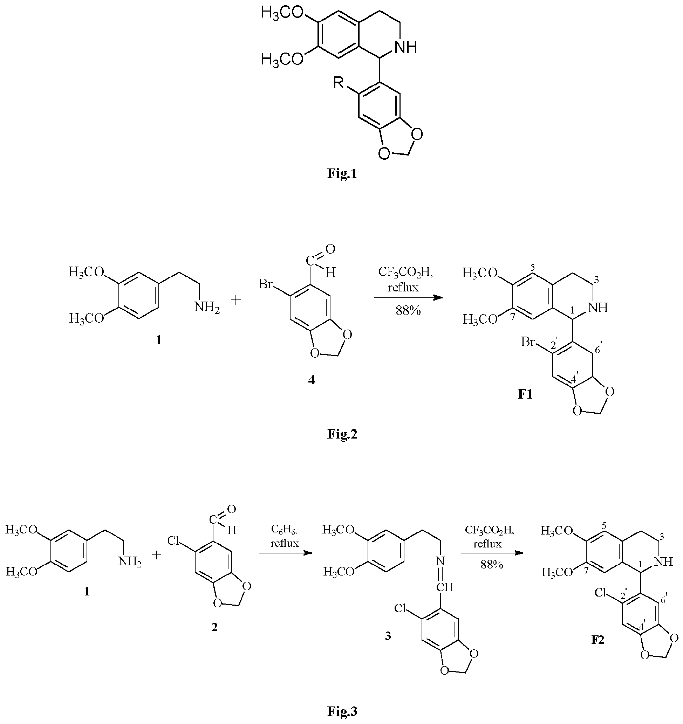

## Claims

1. Use of a compound having structural formula: 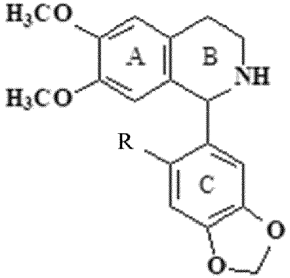 wherein R represents Br or Cl,
as agents that have cytotoxic activity against CCRF-CEM cells (T-lymphoblastic leukemia) and exhibit an antitumor effect against various types of leukemia - T-acute lymphoblastic leukemia (T-ALL), B-acute lymphoblastic leukemia (B-ALL), acute lymphoblastic leukemia (ALL), acute myeloblastic leukemia (AML), acute myelomonoblastic leukemia (AMML) and acute monocytic leukemia (AML).

2. Use according to claim 1, *characterized that* the compound is (1-(2'-bromo-4',5'-methylenedioxyphenyl)-6,7-dimethoxy-1,2,3,4-tetrahydroisoquinoline).

3. Use according to claim 1, *characterized that* the compound is (1-(2'-chloro-4',5'-methylenedioxyphenyl)-6,7-dimethoxy-1,2,3,4-tetrahydroisoquinoline).
